# EUROPÄISCHE PATENTANMELDUNG

(11) **EP 0 864 655 A1**
(43) Veröffentlichungstag der Anmeldung: **16.09.1998**
(21) Anmeldenummer: 98100613.3
(22) Anmeldetag: 15.01.1998
(51) Int. Cl.: C12Q 1/68

(54) **Verfahren zur Quantifizierung von Bakterien**

(30) Priorität: 11.03.1997 DE 19709881
(71) Anmelder: HÜLS AKTIENGESELLSCHAFT, 45764 Marl (DE)
(72) Erfinder: Kunz, Roland, 45770 Marl (DE); Gersonde, Klaus, Prof. Dr., 52074 Aachen (DE); Hill, Frank, Dr., verstorben (DE)

(57) **Zusammenfassung**

Die Erfindung betrifft ein Verfahren zur Quantifizierung von Bakterien, wobei
a) die DNA der Bakterien mit einem nicht natürlichen Analogon einer Nukleotidbase markiert wird,
b) die Bakterien aus Stufe a) lysiert werden,
c) die DNA der lysierten Bakterien denaturiert wird,
d) ein enzym-gekoppelten Antikörper oder ein enzym-gekoppeltes Antikörperfragment gegen das nicht natürliche Analogon einer Nukleotidbase zugesetzt wird und
e) nach Abtrennen der nicht an das nicht natürliche Analogon einer Nukleotidbase gebundenen Antikörper oder Antikörperfragmente, die gebundenen, enzymgekoppelten Antikörper oder enzym-gekoppelten Antikörperfragmente als Maß für die Bakterienanzahl bestimmt werden.

## Beschreibung

Die Erfindung betrifft ein Verfahren zur Quantifizierung von Bakterien durch nichtradioaktive Markietung der DNA.

Bakterien sind vielfach Ursache für Infektionen bei Lebewesen, für den Verderb von Nahrungsmitteln und für das Biofouling in technischen Prozessen. In der Biotechnologie werden Bakterien häufig als Nützlinge eingesetzt. Bei mikrobiologischen Untersuchungen ist die Quantifizierung von Bakterien, sowie die Bestimmung ihrer Proliferationsfähigkeit von zentraler Bedeutung. Diese Untersuchung können zur Vermeidung der schädlichen Wirkung der Bakterien dienen, aber auch zur Optimierung ihrer Nutzwirkung.

Es ist bekannt, daß Bakterien durch Ausplattieren auf Nähragar, Bebrüten und Auszählen der einzelnen Kolonien gezählt werden können Dieses Verfahren ist jedoch nur bei einer Gesamtzahl von unter etwa 300 Bakterien praktikabel, weil sonst einzelne Kolonien überlappen können und ein korrekter Zählvorgang nicht mehr möglich ist. Im Regelfall müssen deshalb Verdünnungen über mehrere Größenordnungen vorgenommen werden, die stark fehlerbehaftet sind. Besonders bei der für die Untersuchung von Fouling-Prozessen wichtigen Zahl der auf einer Oberfläche adhärierenden Bakterien kommt als zusätzliche Fehlerquelle das Ablösen der Bakterien von der Oberfläche hinzu.

Verfahren, die direkt für Oberflächen geeignet sind, wie das licht- oder elektronenoptische Auszählen, liefern den gewünschten integralen Wert der Bakterienzahl nur durch Mittelwertbildung über die gesamte Oberfläche, also nach statistischer Auswertung einer großen Anzahl von Zählfeldern. Sie sind deshalb langsam und teuer.

Die Bestimmung von Bakterien über ihren ATP-Gehalt (Ludwicka A. et al., Journal of Microbiological Methods 4, (1985), 169-177) ist ein Verfahren, das nur ungenau auf die Bakterienzahl rückschließen laßt, da der ATP-Gehalt eines Bakteriums innerhalb eines Generationszyklus schwankt.

Die Markierung der Bakterien über den Einbau von tritiummarkierten Thymidin in die DNA birgt die Gefahren des Umgangs mit radioaktiven Stoffen im Labor und bereitet außerdem Entsorgungsprobleme. Weiterhin wird bei diesem Verfahren bei längeren Inkubationszeiten und höheren Dosen von tritiummarkierten Thymidin eine zunehmende Fragmentierung der DNA beobachtet (E. Solary et al., Experimental Cell Research 203 (1992), 495-498).

Die Markierung und Detektion mit Bromdesoxyuridin, wie sie in DE-OS 43 19 506 beschrieben wurde, läßt sich nur auf eukaryontische Zellen, nicht aber auf Bakterien anwenden.

Der Erfindung liegt die Aufgabe zugrunde, ein Verfahren zur Quantifizierung von Bakterien zu entwickeln, welches einen nicht mit dem Generationszyklus der Bakterien variierenden Parameter verwendet und auch bei Proben mit hoher räumlicher Inhomogenität der Bakterienverteilung einen integralen Wert liefert.

Es wurde überraschenderweise gefunden, daß Bakterien mit einem nicht natürlichen Analogon einer Nukleotidbase markiert und Antikörper oder Antikörperfragmente nach Koppeln an das nicht natürliche Analogon einer Nukleotidbase als Maß für die Bakterienanzahl bestimmt werden können.

Gegenstand der vorliegenden Erfindung ist daher ein Verfahren zur Quantifizierung von Bakterien, dadurch gekennzeichnet, daß
a) die DNA der Bakterien mit einem nicht natürlichen Analogon einer Nukleotidbase markiert wird,
b) die Bakterien aus Stufe a) lysiert werden,
c) die DNA der lysierten Bakterien denaturiert wird,
d) ein enzym-gekoppelten Antikörper oder ein enzym-gekoppeltes Antikörperfragment gegen das nicht natürliche Analogon einer Nukleotidbase zugesetzt wird und
e) nach Abtrennen der nicht an das nicht natürliche Analogon einer Nukleotidbase gebundenen Antikörper oder Antikörperfragmente, die gebundenen, enzym-gekoppelten Antikörper oder enzym-gekoppelten Antikörperfragmente als Maß für die Bakterienanzahl bestimmt werden.

Das erfindungsgemäße Verfahren ermöglicht eine einfache Quantifizierung von Bakterien ohne die Verwendung von radioaktiven Substanzen.

Mit dem erfindungsgemäßen Verfahren kann die in üblicher Weise metabolisch markierte DNA auf einfache Weise zur Quantifizierung von Bakterien benutzt werden, wenn man zuvor eine Kalibrationskurve aufnimmt z. B. durch Ausplattieren oder mikroskopisches Auszählen der Bakterien.

Die Markierung der DNA erfolgt durch den Einbau eines beliebigen Analogons von natürlichen DNA-Biosynthese-Vorstufen, sofern dieses Analogon im gleichen Maße wie eine natürliche DNA-Biosynthese-Vorstufe in die DNA eingebaut wird und ein Antikörper gegen dieses Analogon verfügbar ist.

Als nicht natürliches Analogon einer Nukleotidbase kann 5-Brom-2'desoxyuridin eingesetzt werden.

Geeigneterweise erfolgt die Markierung der DNA durch Aufzucht der Bakterien in einem Kulturmedium, das 5-Brom-2*'*-Desoxyuridin in einer Konzentration von 10 µmol/l enthält.

Im erfindungsgemäßen Verfahren können die Bakterien an einer Oberfläche adheriert vorliegen. Die Immobilisierung der Bakterien kann beispielsweise durch Inkubation von Kunststoffen wie Polyethylen, Polystyrol oder Polyurethan erfolgen

Weiterhin können die Bakterien durch Zentrifugation, gegebenenfalls mit einem Trägermaterial, quantitativ immobilisiert werden.

Die Zellwand der Bakterien wird daraufhin lysiert Die Lyse der Bakterien kann mit einer starken Säure oder mit Lysozym oder einem anderen lytischen Enzym erfolgen.

Der Einsatz von Lysozym-Lösung in einer Konzentration von etwa 70 000 U/ml hat sich besonders bewährt.

Dann wird in der dem Fachmann bekannten Art und Weise die DNA der immobilisierten Bakterien denaturiert. Die Denaturierung kann durch Erhitzen, z. B. durch Mikrowellen oder durch Behandlung mit einem Enzym, beispielsweise Exonuclease III erfolgen. Anschließend wird ein markierter Antikörper oder ein markiertes Antikörperfragment gegen das in die DNA eingebaute, nicht natürliche Analogon einer Nukleotidbase oder dessen fünktionelles Fragment an die markierte DNA gebunden. Dieser Antikörper oder dieses Antikörperfragment kann entweder mit einem Enzym oder einem Farbstoff direkt markiert sein, oder durch einen entsprechend markierten weiteren Antikörper markiert werden.

Wird als nicht natürliches Analogon einer Nuleotidbase 5-Brom-2'-desoxyuridin eingesetzt, so eignet sich als Antikörper beispielsweise ein Peroxidase-gekoppelter Antikörper oder dessen Fragment.

Die Quantifizierung der Bakterien erfolgt z. B. durch UV-Absorptionsmessung des an die denaturierte DNA gebundenen Antikörpers, oder durch Farbreaktion eines an den Antikörper gekoppelten Enzyms.

Die nachfolgenden Beispiele sollen die Erfindung näher erläutern.

### Beispiel 1: Quantifizierung von Bakterien in Suspension

Bakterien des Stammes Klebsiella pneumoniae (DSM 798) wurden 17 Stunden bei 37°C unter Schütteln in einem Medium inkubiert, das neben jeweils 10 g/l Hefelysat, Caseinpepton und Glucose 10 µmol/l 5-Brom-2*'*-desoxyuridin (BrdU) enthielt. Nach mehrmaliger Zentrifugation, Verwerfen des Überstandes und Aufnehmen in Hefelysat/Caseinpepton/Glucose-Medium konnten die so gewonnen markierten Bakterien ohne Veronreinigung mit freiem BrdU erhalten werden. Zum Erstellen einer Kalibrierkurve wurde eine Verdünnungsfreihe angefertigt, je 1 ml der Suspensionen unterschiedlicher Konzentration zentrifugiert und der Überstand verworfen. Die so erhaltenen Pellets wurden 15 Minuten bei 50 °C getrocknet und anschließend mit 1 ml einer Lysozymlösung aus Hühnereiweiß (Fluka AG 62971, 1 mg/ml in Phosphatgepufferter Saline, PBS) für 15 Minuten inkubiert. Der Überstand wurde verworfen und anschließend mit 200 µl einer FixDenat Lösung (Boehringer Mannheim GmbH) für 30 Minuten bei Raumtemperatur inkubiert. Der Überstand wurde verworfen und die Pellets getrocknet. Dann wurde 90 Minuten bei Raumtemperatur mit 100µl eines Peroxidase-gekoppelten Antikörper Fab-Fragmentes gegen BrdU-DNA (Boehringer Mannheim GmbH) inkubiert, der Überstand verworfen und 3 mal mit je 200 µl PBS gewaschen. Dann wurde je 200 µl Tetramethylbenzidin in einer essigsauren Lösung von Wasserstoffperoxid als Substrat zugegeben, 30 Minuten bei Raumtemperatur inkubiert und in 800 µl einer 0.1 mol/l Schwefelsäure-Lösung aufgenommen und bei 450 nm die Extinktion bestimmt (Referenzwellenlänge 690 nm, Schichtdicke 1 cm).

**Tabelle 1**

| Quantifizierung markierter Bakterien | |
|---|---|
| Anzahl der markierten Bakterien | E (450 nm) - E (690 nm) |
| 10 000 | 1.648 |
| 6 000 | 1.317 |
| 3 000 | 0.972 |
| 1 000 | 0.805 |

### Beispiel 2: Quantifizierung adhärenter Bakterien

Prüfmaterialien waren runde Folienstücke der Referenz-Biomaterialien der Europäischen Gemeinschaft (EUROBIOMAT Forschungsprogramm, Berlin) Polyurethan, Polyethylen, Polypropylen sowie eine Folie aus Polyamid 12 der Hüls AG. Von letzterem wurde auch ein durch eine bakterienabweisende Beschichtung gemäß DE-A 197 00 080.0 oberflächenmodifiziertes Exemplar untersucht. Der Durchmesser der Folienstücke betrug jeweils 1.4 cm.

Als Bakterienstamm diente Staphylococcus aureus (klinisches Isolat, Hygieneinstitut Gelsenkirchen). Durch 16-stündige Inkubation bei 37 °C in Hefelysat/Caseinpepton/Glucose-Medium gemäß Beispiel 1 mit 6 µmol/l BrdU, Zentrifugation, Waschen und Aufnehmen des Pellets in steriler PBS wurde eine Suspension von 10⁸ Bakterien/ml erhalten. Als Negativ-Kontrolle diente eine identisch behandelte Staphylococcus aureus Kultur ohne Inkubation mit BrdU.

Die Folienstücke wurden in je 1 ml der Bakteriensuspension für 2 Stunden bei Raumtemperatur inkubiert und anschließend durch 3-maliges Eintauchen in sterile PBS gewaschen. Die adhärenten Bakterien wurden 15 Minuten in 1 ml einer Lysozymlösung (1mg/ml in PBS) inkubiert. Die Lösung wurde abgesaugt und die Folienstücke zur Denaturierung der bakteriellen DNA 30 Minuten mit 250 µl einer Fix-Denat Lösung behandelt, abgesaugt und getrocknet. Schließlich wurde 90 Minuten mit 250 µl einer Lösung von Peroxidase-markiertem Antikörper gegen BrdU-DNA inkubiert. Nach 3-maligem Waschen mit PBS wurden 200 µl Tetramethylbenzidin-Lösung gemäß Beispiel 1 zugegeben und 15 Minuten bei Raumtemperatur inkubiert. Die farberzeugende Reaktion wurde durch Überführen in eine 0.1 mol/l Schwefelsäure-Lösung gestoppt und dann am Photometer bei 450 nm vermessen. Die Referenzwellenlänge betrug 690 nm. Zum Aufstellen einer Kalibrierkurve wurde die Anzahl der Bakterien auf den Folienstücken durch Auszählen Rasterelektronenmikroskopischer Aufnahmen bestimmt.

**Tabelle 2**

| Quantifizierung Oberflächen-adhärenter, markierter Bakterien | | |
|---|---|---|
| Material | Adhärente Zellen | E (450 nm) - E (690 nm) |
| Polyamid 12 | 1.2*10⁶ | 0.852 |
| Polyurethan | 5*10⁶ | 0.969 |
| Polyethylen | 4*10⁵ | 0.732 |
| Polypropylen | 1*10⁶ | 0.801 |
| Polyamid 12 bakterienabweisend modifiziert | 3*10³ | 0.356 |

## Patentansprüche

1. Verfahren zur Quantifizierung von Bakterien,
dadurch gekennzeichnet,
daß
a) die DNA der Bakterien mit einem nicht natürlichen Analogon einer Nukleotidbase markiert wird,
b) die Bakterien aus Stufe a) lysiert werden,
c) die DNA der lysierten Bakterien denaturiert wird,
d) ein enzym-gekoppelten Antikörper oder ein enzym-gekoppeltes Antikörperfragment gegen das nicht natürliche Analogon einer Nukleotidbase zugesetzt wird und
e) nach Abtrennen der nicht an das nicht natürliche Analogon einer Nukleotidbase gebundenen Antikörper oder Antikörperfragmente, die gebundenen, enzymgekoppelten Antikörper oder enzym-gekoppelten Antikörperfragmente als Maß für die Bakterienanzahl bestimmt werden.

2. Verfahren nach Anspruch 1,
dadurch gekennzeichnet,
daß die Bakterien an einer Oberfläche adhäriert vorliegen.

3. Verfahren nach den Ansprüchen 1 und 2,
dadurch gekennzeichnet,
daß als nicht natürliches Analogon einer Nukleotidbase 5-Brom-2'-desoxyuridin eingesetzt wird.

4. Verfahren nach den Ansprüchen 1 bis 3,
dadurch gekennzeichnet,
daß die Lyse der Bakterien mit Lysozym, oder einer starken Säure oder einem anderen lytischen Enzym erfolgt.
